# EUROPEAN PATENT SPECIFICATION

(11) **EP 0 338 670 B1**
(45) Date of publication and mention of the grant of the patent: **11.05.1994**
(21) Application number: 89302340.8
(22) Date of filing: 09.03.1989
(51) Int. Cl.: A61M 11/00, A61K 9/00, B65D 83/14

(54) **Pharmaceutical Compositions**
Pharmazeutische Mischungen
Composés pharmaceutiques

(30) Priority: 22.03.1988 GB 8806818; 16.08.1988 GB 8819490
(43) Date of publication of application: 25.10.1989
(60) Divisional application: 93202207.2
(73) Proprietor: FISONS plc, Ipswich Suffolk IP1 1QH (GB)
(72) Inventor: Chawla, Brinda Paul Singh, West Bridgford Nottingham (GB); Clark, Andrew Reginald, Loughborough Leicestershire (GB); Dean, Desmond Alfred, Beeston Nottingham (GB)
(74) Representative: Wright, Robert Gordon McRae

(56) References cited:
- EP-A- 0 143 577
- EP-A- 0 260 067
- EP-A- 0 274 590
- US-A- 3 024 787
- US-A- 3 131 834
- US-A- 3 985 868

## Description

This invention relates to pharmaceutical compositions, in particular compositions for administration by inhalation, and the use of such compositions in the treatment of reversible obstructive airways disease.

The administration of medicaments by inhalation is well known. Medicaments for administration by the inhalation route are generally formulated either as powders, for administration by insufflation or as pressurised aerosols, or as solutions, eg aqueous solutions, for administration by nebulisation.

Solutions for inhalation may be put up either as single dose or as multi-dose formulations. Both presentations suffer from certain disadvantages. Single dose packaging is less convenient to use and more expensive and wasteful to manufacture. Such packaging frequently takes the form of glass ampoules, each containing a unit dose. Typically, the ampoule is broken open immediately prior to use and the contents transferred to a nebuliser for administration. The breaking open of the ampoule can give rise to the formation of glass sherds which are liable to be inhaled by the patient. This is clearly undesirable. Multi-dose packaging, in which the solution is packaged as a bulk from which unit doses can be dispensed immediately prior to use, is on the face of it much more attractive. However, it has hitherto been found that, in order to maintain the sterility of the solution, the formulation must contain a preservative. This is a serious disadvantage since any preservative used may inter alia adversely affect the stability of the solution or, more seriously, may cause unwanted side-effects. In particular, the preservative most commonly used, benzalkonium chloride, has been found to have unacceptable sensitising, i.e. allergic, effects or to have bronchoconstrictor properties when administered directly to the lung.

A preferred solution contains sodium cromoglycate and chlorbutol. This solution is particularly advantageous in that, although it contains a preservative, it can be administered to a patient's lung with no significant sensitising or bronchodilatory effect.

European Patent Application Number 274590 filed before but published after the priority date of the present invention and having the Netherlands as designated state in common with the present patent, discloses solutions containing cromoglycic acid, chlorbutol and sorbitol for use as eyedrops.

EP-A-274590 describes the dissolution of chlorbutol and sorbitol in a 1.5 times strength solution of sodium cromoglycate followed by dilution with distilled water.

Conventional sterile formulations of sodium cromoglycate are prepared by making a double strength solution of the preservative, e.g. benzalkonium chloride, and a double strength solution or sodium cromoglycate and mixing the two together. However, we have now found that this conventional method of preparation is not suitable for aqueous solutions of sodium cromoglycate and chlorbutol.

According to the invention, therefore, we provide a method for the preparation of an aqueous solution of sodium cromoglycate and chlorbutol which comprises dissolving chlorbutol in distilled water at a temperature in the range 20 - 60 °C in a sealed or covered vessel and admixing the resulting solution with solid sodium cromoglycate.

We prefer the chlorbutol to be dissolved at a temperature in the range 45-55 °C, more preferably at a temperature of about 50 °C.

The concentration of chlorbutol in the solution should be such that bacterial growth in the formulation is inhibited. We have found that acceptable concentrations of chlorbutol are greater than 0.25% w/v but that the upper limit for the concentration of chlorbutol is about 0.6% w/v.

The concentration of sodium cromoglycate in the solution may be in the range 0.1 to 10%, preferably from 0.5 to 5% and more preferably about 1 or 2% w/v.

The solution may also contain an effective proportion of a pharmaceutically acceptable chelating agent or sequestering agent such as ethylene diamine tetraacetic acid or its salts, e.g. its disodium salt (disodium edetate).

The concentration of the chelating agent or sequestering agent should be such as to ensure that no precipitation of metal salts of cromoglycic acid occurs. A suitable concentration may be from 0.1 to 1.0% w/v and preferably from 0.04 to 0.06% w/v, e.g. 0.05% w/v.

The formulation may also contain buffers, e.g. sodium dihydrogen orthophosphate (sodium acid phosphate BP), disodium hydrogen phosphate (sodium phosphate BP), sodium citrate/citric acid, and boric acid/sodium borate.

The formulation preferably has a pH in the range 3.0 to 6.0, more preferably 4.0 to 5.0.

The formulation may be isotonic with physiological fluids by the incorporation of a suitable tonicity agent, e.g. sodium chloride.

The solution of sodium cromoglycate and chlorbutol is useful, inter alia, in the treatment of reversible obstructive airways disease, including "extrinsic" allergic asthma and "intrinsic" asthma (in which no sensitivity to extrinsic antigen can be demonstrated).

According to another aspect of the invention there is provided the use of sodium cromoglycate and chlorbutol in the manufacture of a medicament for the treatment of reversible obstructive airways disease.

An aliquot of solution may be administered as a nebulised cloud which may be produced by known techniques, eg using a conventional nebuliser.

The dosage to be administered will vary with the particular inhalation medicament used, and with the condition to be treated and its severity. However, in general for sodium cromoglycate a total daily dose of active ingredient in the range 15 to 30mg, eg 20mg, is satisfactory. The total daily dose may be given in 1 to 4 divided doses.

Suitable unit volumes to be administered are in the range 1 to 4ml, eg about 2ml. The unit volume is preferably administered 4 times a day or as required by the patient.

The formulation are preferably packaged in a multidose dispenser containing 10 to 300ml of solution. We prefer the formulations to be packaged in 60ml, 120ml or 240ml volumes.

The invention is illustrated, but in no way limited, by the following Examples.

### Example 1

| Formulation 1 | |
|---|---|
| Sodium cromoglycate | 1.00 % w/v |
| Chlorbutol | 0.50 |
| Disodium edetate | 0.05 |

80ml distilled water was added to 0.5g chlorbutol in a volumetric flask. The flask was sealed and placed in an ultrasonic water bath for one day at a temperature of 25-40°C. 1g sodium cromoglycate and 0.05g disodium edetate were added to this solution and the volume made up to 100ml with distilled water.

| Formulation 2 | |
|---|---|
| Sodium cromoglycate | 1.00 % w/v |
| Chlorbutol | 0.50 |

This formulation was prepared following the method used for Formulation 1 above.

### Stability

Formulations 1 and 2 were stored at 4°C, 25°C and 37°C and analysed at intervals of 1 week, 2 weeks, 4 weeks, 2 months and 3 months using high performance liquid chromatography (HPLC). The formulations were found to be stable at all temperatures and times.

### Example 2

Aliquots of a bulk quantity of Formulation 1, prepared as described in Example 1, were sterile-filled into epoxy-lined aluminium cans fitted with metering valves (Lablabo, Montrouge, France) with a 2ml metering volume. Three sizes of can were used with capacities of approximately 60, 120 and 240 ml. The cans were then pressurised with nitrogen gas to a pressure of approximately 70 kPa.

The filled cans were then subjected to a stability test program at 4°C/ambient relative humidity and at 25°C/ambient relative humidity, and 120ml cans were also stored at 40°C/75% relative humidity. Some cans were also cycled between 4°C and 40°C to simulate changes that may be encountered during shipping and handling.

Various parameters were tested prior to storage, including:
1. Appearance/odour
2. pH
3. Microbial count and absence of pathogens
4. Sodium cromoglycate concentration
5. Sodium cromoglycate related substances concentration
6. Chlorbutol concentration
7. Disodium edetate concentration
8. Pressure
The cycled samples, samples stored at 40°C/75%RH for 1, 2 and 3 months and samples stored at 25°C/ambient RH up to 6 months were also tested.

All initial and subsequent test results were within their respective specifications except for the presence of an expected small amount of precipitate in samples stored at 40°C/75%RH after 3 months. This precipitate is probably cromoglycic acid formed as a result of temperature and drop in pH resulting from the breakdown of a small amount of chlorbutol at the high temperature after 3 months storage.

## Claims

1. A process for the preparation of an aqueous solution of sodium cromoglycate and chlorbutol which comprises dissolving chlorbutol in distilled water at a temperature in the range 20 - 60 °C in a sealed or covered vessel and admixing the resulting solution with solid sodium cromoglycate.

2. The use of sodium cromoglycate and chlorbutol in the manufacture of a medicament for the treatment of reversible obstructive airways disease.

## Patentansprüche

1. Verfahren zur Herstellung einer wässerigen Lösung von Natriumcromoglycat und Chlorbutol umfassend Lösen von Chlorbutol in destillierten Wasser bei einer Temperatur im Bereich von 20 - 60°C in einem verschlossenen oder bedeckten Gefäß und Mischen der erhaltenen Lösung mit festem Natriumcromoglycat.

2. Verwendung von Natriumcromoglycat und Chlorbutol bei der Herstellung eines Medikaments zur Behandlung reversibler obstruktiver Atemwegserkrankungen.

## Revendications

1. Procédé pour la préparation d'une solution aqueuse de cromoglicate de sodium et de chlorbutol qui comprend la dissolution du chlorbutol dans de l'eau distillée à une température dans l'intervalle de 20 à 60°C dans un récipient scellé ou couvert et le mélange de la solution résultante avec le cromoglicate de sodium.

2. Utilisation de cromoglicate de sodium et de chlorbutol dans la fabrication d'un médicament pour le traitement de maladies de l'obstruction des voies respiratoires réversibles.
